# EUROPEAN PATENT SPECIFICATION

(11) **EP 2 519 202 B1**
(45) Date of publication and mention of the grant of the patent: **27.04.2016**
(21) Application number: 10784352.6
(22) Date of filing: 02.11.2010
(51) Int. Cl.: A61F 13/534, A61F 13/539

(54) **ABSORBENT ARTICLE**
SAUGFÄHIGER ARTIKEL
ARTICLE ABSORBANT

(30) Priority: 28.12.2009 JP 2009298704
(43) Date of publication of application: 07.11.2012
(73) Proprietor: Livedo Corporation, Shikokuchuo-shi Ehime 799-0122 (JP)
(72) Inventor: URUSHIHARA, Makiko, Mima-gun Tokushima 779-4104 (JP)
(74) Representative: Müller-Boré & Partner Patentanwälte PartG mbB
(86) International application number: PCT/JP2010/006467
(87) International publication number: WO 2011/080858

(56) References cited:
- EP-A1- 1 609 448
- EP-A1- 1 632 206
- EP-A2- 1 116 479
- WO-A1-97/34558

## Description

### Technical Field

The present invention relates to an absorbent article such as an incontinence pad (including a light incontinence pad), a sanitary napkin, and a disposable diaper.

### Background Art

Conventionally, there is known an absorbent article comprising a sheet-shaped absorbent layer which contains an absorbent polymer but does not contain a pulp fiber between nonwoven fabric sheets. For example, Patent Literature 1 discloses an absorbent article comprising a laminate sheet in which two or more of the sheet-shaped absorbent layers are laminated. Patent Literature 2 discloses an absorbent laminate comprising the sheet-shaped absorbent layer and a fiber assembly layer which is disposed under the sheet-shaped absorbent layer, that is a back sheet side, and which contains an absorbent polymer and pulp fibers.

### Citation List

### Patent Literature

PTL 1: Japanese Patent Application Laid-Open Publication No. 2004-313580
PTL 2: Japanese Patent Application Laid-Open Publication No. 2004-275225

### Summary of Invention

### Technical Problem

The sheet-shaped absorbent layer does not contain pulp fibers, and thus can be formed so as to be thin while maintaining its absorptive capacity. However, the absorption rate of an absorbent polymer is lower than that of pulp fibers, and therefore, when a bodily fluid such as urine is excreted in a large amount at one time, there is the possibility that the bodily fluid is not rapidly absorbed. In particular, when the two or more sheet-shaped absorbent layers are laminated as in the absorbent article disclosed in Patent Literature 1, the absorptive capacity of the absorbent article is increased, but there is the possibility that it is less likely to absorb a bodily fluid rapidly. In addition, when the sheet-shaped absorbent layer and the fiber assembly layer are laminated as in the absorbent article disclosed in Patent Document 2, it is likely to be difficult to increase the absorptive capacity while the absorbent article is formed so as to be thin.

WO 97/34558 A1 discloses absorbent components having a fluid acquisition zone formed by a core which contains swellable fluid storage material.

EP 1 609 448 A1 discloses a disposable absorbent article in which an absorbent mat includes a sheet-shaped water-absorbent layer that contains a water-absorbent resin powder but does not contain pulp fibers; and a fiber assembly layer that contains a water-absorbent resin powder and pulp fibers.

The present invention has been achieved in view of the above circumstances, and an object of the present invention is to provide an absorbent article which can absorb a bodily fluid such as urine rapidly, and can be formed so as to be thin while having a high absorptive capacity.

### Solution to Problem

An absorbent article of the present invention which solves the above problems comprises a top sheet, a back sheet and an absorbent laminate disposed between the top sheet and the back sheet, wherein: the absorbent laminate comprises an upper sheet-shaped absorbent layer, a fiber assembly layer and a lower sheet-shaped absorbent layer provided in this order from the top sheet side; the fiber assembly layer contains pulp fibers; and each of the upper sheet-shaped absorbent layer and the lower sheet-shaped absorbent layer contains an absorbent polymer but does not contain a pulp fiber between nonwoven fabric sheets. The absorbent article of the present invention can absorb a bodily fluid rapidly even when the bodily fluid are excreted in a large amount at one time. That is, even when the upper sheet-shaped absorbent layer does not fully absorb a bodily fluid, which is excreted in a large amount at one time, the fiber assembly layer can absorb the bodily fluid rapidly. The bodily fluid diffuses in the fiber assembly layer, and therefore, the bodily fluid which has passed through the fiber assembly layer comes to be absorbed by the lower sheet-shaped absorbent layer in a broad area, whereby rapid absorption of the bodily fluid by the lower sheet-shaped absorbent layer is achieved. In addition, according to the absorbent article of the present invention, since both of the upper sheet-shaped absorbent layer and the lower sheet-shaped absorbent layer can absorb and fix a bodily fluid therein, the absorbent article can be formed so as to be thin while having a high absorptive capacity.

Each of the upper sheet-shaped absorbent layer and the lower sheet-shaped absorbent layer has a plurality of absorbent polymer present regions, in each of which the absorbent polymer is provided, and an absorbent polymer absent region adjacent to the absorbent polymer present region between the nonwoven fabric sheets; and the nonwoven fabric sheets are joined together at the absorbent polymer absent region to form a sealing portion. When the absorbent polymer absent regions are disposed in the upper sheet-shaped absorbent layer and the lower sheet-shaped absorbent layer, and the nonwoven fabric sheets are joined together at these regions to form the sealing portions, a bodily fluid easily pass through the absorbent polymer absent region. Therefore, in the upper sheet-shaped absorbent layer, when a bodily fluid are excreted in a large amount at one time, the bodily fluid easily transfers to the fiber assembly layer through the absorbent polymer absent region, and thus are rapidly absorbed by the absorbent laminate. In the lower sheet-shaped absorbent layer, a part of the bodily fluid which has transferred to the lower sheet-shaped absorbent layer easily transfers to the lower side (the back sheet side) of the lower sheet-shaped absorbent layer through the absorbent polymer absent region. As a result, the bodily fluid is easily absorbed from both the upper side and the lower side of the lower sheet-shaped absorbent layer, whereby rapid absorption of the bodily fluid by the lower sheet-shaped absorbent layer is achieved.

The nonwoven fabric sheets of at least one of the upper sheet-shaped absorbent layer and the lower sheet-shaped absorbent layer may be partly heat-sealed at the absorbent polymer absent region. According to this constitution, a bodily fluid easily spreads at a heat-sealed portion, which is formed by heat-sealing the upper or lower sheet-shaped absorbent layer, in the planar direction of the upper or lower sheet-shaped absorbent layer, whereas at a non-heat-sealed portion of the absorbent polymer absent region, a bodily fluid easily pass through the upper or lower sheet-shaped absorbent layer in the thickness direction. Therefore, appropriate adjustment of the proportion of the heat-sealed portion and the non-heat-sealed portion allows optional adjustment of spread and permeation of a bodily fluid in the upper or lower sheet-shaped absorbent layer.

The absorbent polymer present regions are preferably disposed intermittently in the width direction of the absorbent laminate. When the absorbent polymer present regions are disposed in this manner, a bodily fluid easily spreads in the longitudinal direction in the upper or lower sheet-shaped absorbent layer, whereby the bodily fluid is rapidly absorbed by the absorbent laminate.

Preferably, each of the absorbent polymer present regions is disposed in the shape of a practically straight line extending in the longitudinal direction of the absorbent laminate and having a length of 75% or more of the absorbent laminate in the longitudinal direction, and the absorbent polymer present regions are aligned practically parallel each other in the width direction of the absorbent laminate. When the absorbent polymer present regions are provided in this manner, a bodily fluid easily spreads in the longitudinal direction in the upper or lower sheet-shaped absorbent layer, and further, the absorptive capacity of the upper or lower sheet-shaped absorbent layer is easily ensured since the absorbent polymer present regions are disposed so as to have relatively large areas.

Preferably, a maximum distance between the adjacent absorbent polymer present regions of the upper sheet-shaped absorbent layer is larger than that of the lower sheet-shaped absorbent layer. According to this constitution, permeation and surficial spread of a bodily fluid in the upper sheet-shaped absorbent layer is enhanced rather than those in the lower sheet-shaped absorbent layer, and therefore, the absorbent laminate can absorb a bodily fluid more rapidly.

The nonwoven fabric sheets of the upper sheet-shaped absorbent layer or the lower sheet-shaped absorbent layer are preferably kept joined together at the sealing portion when the upper sheet-shaped absorbent layer or the lower sheet-shaped absorbent layer absorbs a bodily fluid. According to this constitution, since the sealing portion are maintained when the absorbent polymer provided in the upper or lower sheet-shaped absorbent layer absorbs a bodily fluid to swell, permeation and spread of a bodily fluid in the upper or lower sheet-shaped absorbent layer is easily ensured.

Preferably, an adhesive is applied to the nonwoven fabric sheets of the upper sheet-shaped absorbent layer and the lower sheet-shaped absorbent layer respectively to form adhesive layers; the absorbent polymer disposed at the absorbent polymer present region is fixed to the nonwoven fabric sheet by the adhesive layer; and the nonwoven fabric sheets are joined together at the absorbent polymer absent region by the adhesive layer. According to this constitution, the absorbent polymer is less likely to move in the upper and lower sheet-shaped absorbent layer, and the absorbent capability of the upper and lower sheet-shaped absorbent layer is sufficiently ensured. In addition, a feeling of discomfort due to unevenly location of the absorbent polymer is less likely to be provided to a wearer.

The fiber assembly layer has an opening. When the fiber assembly layer has the opening, a bodily fluid which has passed through the upper sheet-shaped absorbent layer can transfer directly to the lower sheet-shaped absorbent layer through the opening of the fiber assembly layer, thereby rapidly absorbed by the lower sheet-shaped absorbent layer. On the other hand, at a part of the fiber assembly layer where the opening is not provided, a bodily fluid which has passed through the upper sheet-shaped absorbent layer diffuses in the fiber assembly layer, and therefore, the absorptive capability of the lower sheet-shaped absorbent layer can be utilized in a broad area. As a result, the absorption rate of the absorbent laminate is improved in totally.

### Advantageous Effects of Invention

The absorbent article of the present invention can rapidly absorb a bodily fluid such as urine, and can be formed so as to be thin while having a high absorptive capacity.

### Brief Description of Drawings

[fig.1]Fig. 1 shows an example of a cross-sectional view of an upper sheet-shaped absorbent layer.
[fig.2A]Fig. 2A shows an example of an arrangement pattern of an absorbent polymer present region and an example of a sheet-shaped absorbent layer provided with heat-sealed portions.
[fig.2B]Fig. 2B shows an example of an arrangement pattern of the absorbent polymer present region and an example of the sheet-shaped absorbent layer provided with heat-sealed portions.
[fig.3A]Fig. 3A shows an example of an arrangement pattern of the absorbent polymer present region.
[fig.3B]Fig. 3B shows an example of an arrangement pattern of the absorbent polymer present region.
[fig.3C]Fig. 3C shows an example of an arrangement pattern of the absorbent polymer present region.
[fig.3D]Fig. 3D shows an example of an arrangement pattern of the absorbent polymer present region.
[fig.4]Fig. 4 shows a plan view of an incontinence pad as one embodiment of an absorbent article of the present invention.
[fig.5]Fig .5 shows a cross-sectional view taken along line V-V of the absorbent article shown in Fig. 4.

### Description of Embodiments

An absorbent article of the present invention comprises a top sheet, a back sheet and an absorbent laminate disposed between the top sheet and the back sheet. The top sheet is a sheet which is located on a wearer's side, that is an inner side, when the absorbent article is worn. The material of the top sheet is not restricted as long as it is liquid-permeable. The back sheet is a sheet which is located on an opposite side of a wearer, that is an outer side, when the absorbent article is worn. The material of the back sheet is not restricted as long as it is liquid-impermeable.

The top sheet and the back sheet may be composed of a nonwoven fabric, a woven fabric, a knitted fabric, a plastic film, a laminate of a plastic film and a nonwoven fabric, or the like. Examples of the laminate include a laminate in which a sheet of a nonwoven fabric and a sheet of a plastic film are stacked, and a laminate in which a plastic film is interposed between nonwoven fabrics. When a plastic film or a laminate including a plastic film is used for the top sheet, it is preferable that pores for allowing liquid to permeate through are formed in the plastic film. The top sheet is preferably made of a nonwoven fabric. The back sheet is preferably made of a nonwoven fabric or a plastic film.

When a nonwoven fabric is used for the top sheet or the back sheet, a nonwoven fabric manufactured by a spunbonding method, an air-through method, a point bonding method, a melt blowing method, an airlaid method, a combination of these methods, or the like, is preferably used. Also, a nonwoven fabric manufactured by an SMS method which is a combination of the spunbonding method and the melt blowing method may be used.

When a nonwoven fabric is used for the top sheet or the back sheet, a material of the nonwoven fabric can be selected as appropriate from synthetic fibers such as polypropylene, polyethylene, polyester (e.g., PET) and polyamide; natural fibers such as pulp and silk. Also, composite fibers can be used as the synthetic fibers. Among them, polypropylene, polyethylene, PET, or composite fibers obtained by combining these materials, are preferred. When such a nonwoven fabric is used, a sheet having a high strength and excellent texture is easily obtained.

The absorbent laminate is disposed between the top sheet and the back sheet, and absorbs excrement such as urine. The absorbent laminate comprises an upper sheet-shaped absorbent layer, a fiber assembly layer and a lower sheet-shaped absorbent layer provided in this order from the top sheet side.

In the present invention, "upper" side means a wearer's side when the absorbent article is worn, and "lower" side means the side opposite to a wearer when the absorbent article is worn, that is an outer side. In addition, the direction from the upper side to the lower side is referred to as a thickness direction.

The absorbent laminate has a longitudinal direction and a width direction. The longitudinal direction means a direction extending in a front-back direction at a crotch of a wearer when the wearer wears the absorbent article. The width direction means a direction orthogonal to the longitudinal direction on the same plane as the absorbent laminate. In addition, a direction on the plane formed by the longitudinal direction and the width direction is defined as a planar direction.

A shape (a planar shape) of the absorbent laminate is not particularly limited. The shape of the absorbent laminate is determined as appropriate according to application, and examples of the shape of the absorbent laminate include, for example, a rectangular shape, an hourglass shape, a center nipped-in gourd shape, and a battledore shape.

The fiber assembly layer is explained in the following. The fiber assembly layer is provided between the upper sheet-shaped absorbent layer and the lower sheet-shaped absorbent layer. Preferably, the fiber assembly layer is provided adjacent to both the upper sheet-shaped absorbent layer and the lower sheet-shaped absorbent layer.

The fiber assembly layer contains pulp fibers. Since the fiber assembly layer contains pulp fibers, the fiber assembly layer can rapidly absorb a bodily fluid such as urine which has passed through the upper sheet-shaped absorbent layer. Further, the fiber assembly layer provides excellent diffusion of the absorbed bodily fluid therein, and therefore, the bodily fluid which has passed through the fiber assembly layer is absorbed by the lower sheet-shaped absorbent layer in a broad area, whereby rapid absorption of the bodily fluid by the lower sheet-shaped absorbent layer can achieved.

In the absorbent article of the present invention, the fiber assembly layer is preferably provided for main purposes of rapidly obtaining (absorbing) a bodily fluid and diffusing a bodily fluid therein. Thus, a function of absorbing and fixing a bodily fluid is preferably served mainly by the upper sheet-shaped absorbent layer and the lower sheet-shaped absorbent layer. Therefore, the fiber assembly layer is preferably formed as thin as practical in the condition that obtaining and diffusing a bodily fluid are achieved at a desired level.

As the pulp fibers contained in the fiber assembly layer, split pulp fibers are preferably used. In addition, the split pulp fibers are preferably used in a form of a fiber mass.

As the fiber assembly layer, a shaped product of an absorbent material such as pulp fibers, that is formed into a predefined shape, may be used, or an absorbent material wrapped with a covering sheet such as a paper (e.g., tissue paper) and a liquid-permeable nonwoven fabric may be used.

As the fiber assembly layer, a shaped product obtained by dispersing split pulp fibers in the air and depositing the split pulp fibers on a screen by suction or application of pressure, or a product obtained by wrapping the shaped product with a covering sheet is preferably used. In these cases, an absorbent polymer or thermal-adhesive fibers, which is described below, may be dispersed in the air together with the split pulp fibers, or an absorbent polymer may be applied to the shaped product at a intended location. The thus obtained fiber assembly layer is excellent in shape retaining property and also excellent in absorbency and diffuseness of a bodily fluid.

The fiber assembly layer may contain an absorbent polymer in addition to the pulp fibers. The absorbent polymer is used as a kind of the absorbent material. Examples of the absorbent polymer include polyacrylic acid-based absorbent polymers such as sodium polyacrylate; starch-based absorbent polymers such as a starch-acrylonitrile graft copolymer, a starch-acrylic acid graft copolymer, and a starch-acrylamide graft copolymer; and polyvinyl alcohol-based absorbent polymers such as a crosslinked polyvinyl alcohol. As the absorbent polymer, polyacrylic acid-based absorbent polymers such as sodium polyacrylate are preferably used, since they have a high absorptive capacity. When the fiber assembly layer contains the absorbent polymer, the fiber assembly layer has an enhanced holding capacity of a bodily fluid.

In the present invention, the embodiment that the fiber assembly layer does not contain an absorbent polymer is one of preferred embodiments. When the fiber assembly layer contains pulp fibers and does not contain the absorbent polymer, in particular, a bodily fluid is rapidly absorbed by the fiber assembly layer and broadly diffuses in the fiber assembly layer.

When the fiber assembly layer contains the absorbent polymer, the content ratio of the absorbent polymer in the fiber assembly layer is preferably 40 mass % or less, more preferably 30 mass % or less, and even more preferably 20 mass % or less. When the content ratio of the absorbent polymer exceeds 40 mass %, it is possible that a bodily fluid is less likely to be rapidly absorbed by the fiber assembly layer and the diffusion of a bodily fluid in the fiber assembly layer may be decreased. The lower limit of the content ratio of the absorbent polymer is not particularly specified. That is, the fiber assembly layer may not contain an absorbent polymer. The content ratio of the absorbent polymer may be adjusted as appropriate according to the desired performance of the fiber assembly layer. Here, the content ratio of the absorbent polymer means the content ratio of the absorbent polymer in the absorbent material.

The fiber assembly layer may contain thermal-adhesive fibers such as polyolefin fibers (e.g., polyethylene and polypropylene), polyester fibers (e.g., PET), and polyamide fibers in addition to the pulp fibers. The thermal-adhesive fibers are used as a kind of the absorbent material. When the fiber assembly layer contains these fibers, the fiber assembly layer easily keeps its shape better. The thermal-adhesive fibers may be hydrophilized with a surfactant or the like in order to enhance the affinity for a bodily fluid such as urine.

The fiber assembly layer preferably has a mass per unit area of 90 g/m² or more, more preferably 100 g/m² or more, preferably 150 g/m² or less, and more preferably 140 g/m² or less. When the fiber assembly layer has a mass per unit area in the range of from 90 g/m² to 150 g/m², the fiber assembly layer is not thickened excessively, and the fiber assembly layer which is excellent in absorbency and diffuseness of a bodily fluid is easily obtained.

A shape (a planar shape) of the fiber assembly layer is not particularly limited. Examples of the shape of the fiber assembly layer include, for example, a rectangular shape, an hourglass shape, a center nipped-in gourd shape, and a battledore shape.

The upper sheet-shaper absorbent layer and the lower sheet-shaped absorbent layer are explained in the following. Hereinafter, the upper sheet-shaped absorbent layer and the lower sheet-shaped absorbent layer may be collectively referred to as a sheet-shaped absorbent layer.

The upper sheet-shaped absorbent layer is provided so as to be closer to the top sheet than the lower sheet-shaped absorbent layer and the fiber assembly layer. Therefore, the upper sheet-shaped absorbent layer receives a bodily fluid such as urine, which has passed through the top sheet to reach the absorbent laminate, earlier than the lower sheet-shaped absorbent layer and the fiber assembly layer, basically.

The lower sheet-shaped absorbent layer is provided so as to be closer to the back sheet than the upper sheet-shaped absorbent layer and the fiber assembly layer. The lower sheet-shaped absorbent layer basically receives a bodily fluid such as urine which has passed through the fiber assembly layer.

Each of the upper sheet-shaped absorbent layer and the lower sheet-shaped absorbent layer contains an absorbent polymer but does not contain a pulp fiber between nonwoven fabric sheets. Thus, each of the upper sheet-shaped absorbent layer and the lower sheet-shaped absorbent layer is formed by providing an absorbent polymer between nonwoven fabric sheets, and a pulp fiber is not provided between the nonwoven fabric sheets. Since each of the upper sheet-shaped absorbent layer and the lower sheet-shaped absorbent layer does not contain a pulp fiber between the nonwoven fabric sheets, each layer can be formed so as to be thin while having a high absorptive capacity.

Examples of the absorbent polymer provided in the sheet-shaped absorbent layer include polyacrylic acid-based absorbent polymers, starch-based absorbent polymers, and polyvinyl alcohol-based absorbent polymers, as explained above. Among them, polyacrylic acid-based absorbent polymers such as sodium polyacrylate are preferably used as the absorbent polymer, since they have a high absorptive capacity.

The nonwoven fabric sheets used in the sheet-shaped absorbent layer are liquid-permeable. For such nonwoven fabric sheets, for example, hydrophilic fibers such as cellulose, rayon, and cotton; and hydrophilized hydrophobic fibers such as polypropylene, polyethylene, polyester, and polyamide with a surfactant may be used. When the nonwoven fabric sheets are partly heat-sealed as described below, hydrophilized hydrophobic fibers, such as polypropylene, polyethylene, polyester, and polyamide, with a surfactant are preferably used for the nonwoven fabric sheets, since heat-sealing thereof is facilitated.

In the sheet-shaped absorbent layer, the absorbent polymer may be provided between two nonwoven fabric sheets, that are a first nonwoven fabric sheet on the top sheet side and a second nonwoven fabric sheet on the back sheet side. In this case, each of the first nonwoven fabric sheet and the second nonwoven fabric sheet may be formed of one nonwoven fabric sheet, or may be formed by two or more nonwoven fabric sheets being laminated on each other. Alternatively, concerning the first nonwoven fabric sheet and the second nonwoven fabric sheet, one nonwoven fabric sheet may be folded at a fold line to define two portions across the fold line, one of the two portions may serve as the first nonwoven fabric sheet, and the other of the two portions may serve as the second nonwoven fabric sheet. In this case, the absorbent polymer is provided inside the folded one nonwoven fabric sheet.

A shape (a planar shape) of the sheet-shaped absorbent layer is not particularly limited. Examples of the shape of the sheet-shaped absorbent layer include, for example, a rectangular shape, an hourglass shape, a center nipped-in gourd shape, and a battledore shape. Respective shapes of the upper sheet-shaped absorbent layer and the lower sheet-shaped absorbent layer may be same or different from each other.

In the absorbent article of the present invention, a bodily fluid such as urine which has transferred to the absorbent laminate is absorbed first by the upper sheet-shaped absorbent layer, basically. At this time, when the bodily fluid are excreted in a large amount at one time, the upper sheet-shaped absorbent layer may not fully absorb the bodily fluid, whereby a part of the bodily fluid tends to transfer to the fiber assembly layer. Since the fiber assembly layer has the excellent capability of rapidly absorbing a bodily fluid, the bodily fluid such as urine which has passed through the upper sheet-shaped absorbent layer is rapidly absorbed by the fiber assembly layer. In addition, the bodily fluid which has transferred to the fiber assembly layer diffuses broadly in the fiber assembly layer through voids in the fiber assembly layer. As a result, the bodily fluid which has passed through the fiber assembly layer is absorbed by the lower sheet-shaped absorbent layer in a broad area. Thus, the bodily fluid is rapidly absorbed by the lower sheet-shaped absorbent layer. Therefore, the absorbent article of the present invention can rapidly absorb a bodily fluid such as urine.

According to the absorbent article of the present invention, both of the upper sheet-shaped absorbent layer and the lower sheet-shaped absorbent layer can absorb a bodily fluid to fix therein, and therefore, the absorbent article of the present invention can be formed so as to be thin easily while having a high absorptive capacity.

The absorbent article of the present invention is also excellent in preventing return (wet back) of a bodily fluid to a skin side. Since the upper sheet-shaped absorbent layer contains the absorbent polymer but does not contain a pulp fiber between the nonwoven fabric sheets, even when the upper sheet-shaped absorbent layer absorbs a bodily fluid, the surface thereof is kept relatively dry. On the other hand, when the fiber assembly layer absorbs a bodily fluid, the fiber assembly layer easily becomes wet, and there is the possibility that a part of the absorbed bodily fluid returns by the fiber assembly layer being pressed. However, due to the presence of the upper sheet-shaped absorbent layer provided so as to be closer to the top sheet than the fiber assembly layer, the bodily fluid returning from the fiber assembly layer is easily prevented from reaching the skin of a wearer.

Preferably, each of the upper sheet-shaped absorbent layer and the lower sheet-shaped absorbent layer has a plurality of absorbent polymer present regions, in each of which the absorbent polymer is provided, and an absorbent polymer absent region adjacent to the absorbent polymer present region sandwiched between the nonwoven fabric sheets; and the nonwoven fabric sheets are joined together at the absorbent polymer absent region to form a sealing portion. When the absorbent polymer absent region is disposed in the sheet-shaped absorbent layer, and the nonwoven fabric sheets are joined together at this region to form the sealing portion, a bodily fluid easily pass through the absorbent polymer absent region. Therefore, in the upper sheet-shaped absorbent layer, when a bodily fluid are excreted in a large amount at one time, the bodily fluid easily transfers to the fiber assembly layer through the absorbent polymer absent region, and thus are rapidly absorbed by the absorbent laminate. In the lower sheet-shaped absorbent layer, a part of the bodily fluid which has transferred to the lower sheet-shaped absorbent layer easily permeate the absorbent polymer absent region to reach the lower side (the back sheet side) of the lower sheet-shaped absorbent layer. As a result, the bodily fluid is easily absorbed from both the upper and lower sides of the lower sheet-shaped absorbent layer, whereby rapid absorption of a bodily fluid by the lower sheet-shaped absorbent layer is achieved. Further, the sealing portion serves to spread a bodily fluid in the planar direction on the surface of the sheet-shaped absorbent layer. Therefore, a bodily fluid spread in the planar direction are absorbed by the absorbent polymer or passes through the absorbent polymer absent region, whereby the bodily fluid tends to be absorbed more rapidly by the absorbent laminate.

The sealing portion may be formed by joining the nonwoven fabric sheets together by an adhesive or heat-sealing (thermal fusion-bonding), as described below. The sealing portion also may be formed by ultrasonic-bonding the nonwoven fabric sheets together.

At the sealing portion, the nonwoven fabric sheets are preferably kept joined together when the upper sheet-shaped absorbent layer or the lower sheet-shaped absorbent layer absorbs a bodily fluid. That is, in the upper sheet-shaped absorbent layer, the nonwoven fabric sheets of the upper sheet-shaped absorbent layer are preferably kept joined together at the sealing portion when the upper sheet-shaped absorbent layer absorbs a bodily fluid; and in the lower sheet-shaped absorbent layer, the nonwoven fabric sheets of the lower sheet-shaped absorbent layer are preferably kept joined together at the sealing portion when the lower sheet-shaped absorbent layer absorbs a bodily fluid. When the sheet-shaped absorbent layer absorbs a bodily fluid, the absorbent polymer provided between the nonwoven fabric sheets swells, and therefore, the joining of the nonwoven fabric sheets at the sealing portion may possibly separate. In this case, it may become difficult for a bodily fluid to pass through the absorbent polymer absent region, and the spread of a bodily fluid at the absorbent polymer absent region may be inhibited. Therefore, the nonwoven fabric sheets of the sheet-shaped absorbent layer are preferably kept joined together when the sheet-shaped absorbent layer absorbs.

In order that the joining of the nonwoven fabric sheets is maintained when the sheet-shaped absorbent layer absorbs a bodily fluid, it is preferable that the upper limit of the absorbent polymer content in the absorbent polymer present region is defined. Thus, the content of the absorbent polymer in the absorbent polymer present region is preferably 400 g/m² or less, and more preferably 385 g/m² or less. Meanwhile, in order to ensure sufficient absorption amount in the absorbent polymer present region of the sheet-shaped absorbent layer, the content of the absorbent polymer in the absorbent polymer present region is preferably 100 g/m² or more, and more preferably 150 g/m² or more.

In order that the joining of the nonwoven fabric sheets is maintained when the sheet-shaped absorbent layer absorbs a bodily fluid, it is also preferable that the nonwoven fabric sheets are adhered together by a rubber adhesive or a styrene based-elastomer, or heat-sealed together.

It is preferable that an adhesive is applied to the nonwoven fabric sheets of the upper sheet-shaped absorbent layer and the lower sheet-shaped absorbent layer respectively to form adhesive layers, and the absorbent polymer disposed at the absorbent polymer present region is fixed to the nonwoven fabric sheet by the adhesive layer. The adhesive layer may be disposed on at least one of the nonwoven fabric sheets by which the absorbent polymer is sandwiched; and preferably, the adhesive layers are disposed on both of the nonwoven fabric sheets by which the absorbent polymer is sandwiched. Here, in the absorbent polymer present region, it is sufficient that at least a part of the absorbent polymer provided is fixed to the adhesive layer, and for example, the absorbent polymer in contact with the adhesive layer may be fixed to the adhesive layer. When the absorbent polymer is fixed to the nonwoven fabric sheets by the adhesive layer, the absorbent polymer is less likely to move in the sheet-shaped absorbent layer prior to the absorbent polymer absorbing a bodily fluid, and the absorbent capability of the sheet-shaped absorbent layer is sufficiently ensured. In addition, the absorbent polymer is less likely to be unevenly located in the sheet-shaped absorbent layer since the absorbent polymer is suppressed to move therein, and thus a feeling of discomfort is less likely to be provided to a wearer. Even after the absorbent polymer absorbs a bodily fluid, the gelled absorbent polymer is less likely to move in the sheet-shaped absorbent layer, and as a result, the absorbent polymer is less likely to form a lump to provide a feeling of discomfort to a wearer.

At the absorbent polymer absent region, the nonwoven fabric sheets are preferably joined together by the adhesive layer. In this case, the joining of the nonwoven fabric sheets, that is the sealing portion, is easily maintained, even when the absorbent polymer disposed at the absorbent polymer present region absorbs a bodily fluid to swell.

It is preferred that the adhesive layer does not inhibit absorption and swelling of the absorbent polymer while fixing the absorbent polymer at the absorbent polymer present region. In this respect, the adhesive layer is preferably formed into a net-like structure.

As a method of forming the adhesive layer into a net-like structure, a method for discharging a molten adhesive from a plurality of nozzles in a thread form (a curtain spray method, a spiral coating method or an omega coating method) may be used. In the curtain spray method, for example, a curtain spray coater which comprises: a plurality of small discharge holes arranged linearly; and air injection ports, which are capable of injecting hot air at high speed, provided in the vicinity of each of the discharge holes; may be used. Air is blown off to a molten adhesive discharged from the each discharging hole in a thread form, whereby the adhesive can be applied to a nonwoven fabric as an assembly of nets in which many filamentous adhesives randomly adhere to one another. In the spiral coating method, for example, a spiral spray nozzle gun, in which three or more air injection ports capable of blowing out air in a direction of a center of the nozzle are provided point symmetrically in the vicinity of a hot-melt adhesive discharging hole, may be used. By using the spiral spray nozzle gun, an adhesive layer in which an adhesive filament is formed into a spiral form can be applied to a nonwoven fabric. In the omega coating method, for example, an adhesive layer having an omega-shaped pattern is formed by, while continuously discharging an adhesive from a coating head located above a nonwoven fabric, moving linearly the nonwoven fabric relative to the coating head, and changing the dropping direction of the adhesive by air blow or the like so as to reciprocate substantially perpendicularly to the moving direction of the nonwoven fabric.

As a method of forming the adhesive layer into a net-like structure, it may be employed that a hot-melt adhesive is fell from an adhesive discharging hole in a thread form having a square cross-sectional shape, and applied to a nonwoven fabric in the state that the dropping hot-melt adhesive is laterally waved by applying slit air, which is provided in an acute angle direction relative to the adhesive discharging hole, to the front and back sides of the dropping hot-melt adhesive. According to this method, an adhesive layer having a zig-zag pattern or a meandering pattern is formed on the nonwoven fabric. Alternatively, by a coater method, an adhesive may be applied to a nonwoven fabric in a very thin stripe shape to form the adhesive layer, and the absorbent polymer may be fixed thereto. According to this method as well, the same effect is obtained as in the case of forming the adhesive layer into a net-like structure.

Examples of the adhesive used for the adhesive layer include, for example, rubber adhesives such as natural rubbers, butyl rubbers and polyisoprene; styrene elastomers such as styrene-isoprene-styrene block copolymer (SIS), styrene-butadiene-styrene block copolymer (SBS), styrene-ethylene-butadiene-styrene block copolymer (SEBS), and styrene-ethylene-propylene-styrene block copolymer (SEPS); ethylene-vinyl acetate copolymer (EVA); polyester; acryl elastomers; and polyolefin elastomers. These exemplified adhesives may be used either alone or as a combination of at least two of them. It is preferable that the adhesive has such an adhesive force that the absorbent polymer can be prevented from falling off after absorbing a bodily fluid, and is stretchable to such an extent as to allow swelling of the absorbent polymer. It is also preferable that the adhesive has such an adhesive force that the nonwoven fabric sheets are kept joined together even when the absorbent polymer absorbs a bodily fluid and swells. In these respects, rubber adhesives and styrene elastomers are preferably used.

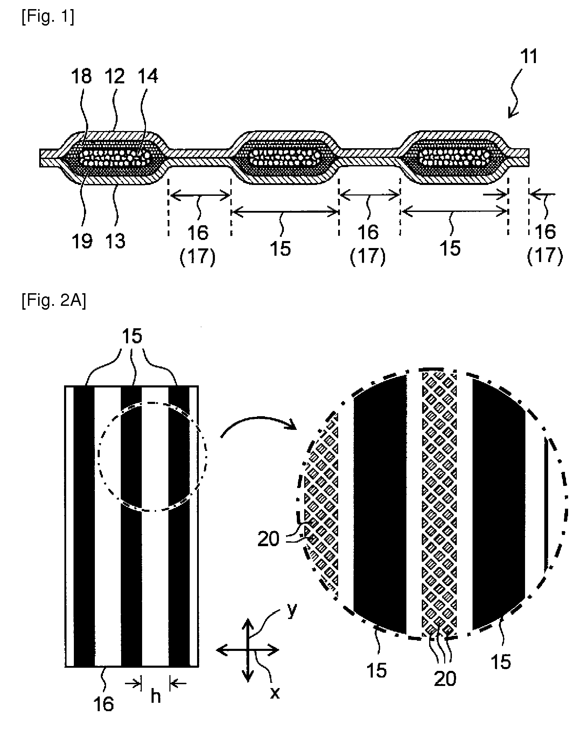

Fig. 1 shows an example of a cross-sectional view of the upper sheet-shaped absorbent layer provided with the adhesive layers. An upper sheet-shaped absorbent layer 11 comprises a first upper nonwoven fabric sheet 12, a second upper nonwoven fabric sheet 13, a first upper adhesive layer 18 formed by applying an adhesive to the first upper nonwoven fabric sheet 12, a second upper adhesive layer 19 formed by applying an adhesive to the second upper nonwoven fabric sheet 13, and absorbent polymers 14 disposed between the first upper adhesive layer 18 and the second upper adhesive layer 19. The upper sheet-shaped absorbent layer 11 has a plurality of absorbent polymer present regions 15 and absorbent polymer absent regions 16 adjacent to the absorbent polymer present region 15. Absorbent polymers 14 of the absorbent polymer present regions 15 are fixed to the first upper nonwoven fabric sheet 12 and the second upper nonwoven fabric sheet 13 by the first upper adhesive layer 18 and the second upper adhesive layer 19, respectively. At the absorbent polymer absent regions 16, the first upper nonwoven fabric sheet 12 and the second upper nonwoven fabric sheet 13 are joined together by the first upper adhesive layer 18 and the second upper adhesive layer 19 to form sealing portions 17. In the above, the upper sheet-shaped absorbent layer is explained with reference to Fig. 1, as an example. In the case of the lower sheet-shaped absorbent layer, the word "upper" in the above description concerning Fig. 1 is replaced by "lower".

It is preferable that the nonwoven fabric sheets of at least one of the upper sheet-shaped absorbent layer and the lower sheet-shaped absorbent layer are partly heat-sealed at the absorbent polymer absent region. When the nonwoven fabric sheets are partly heat-sealed at the absorbent polymer absent region, a bodily fluid easily spreads at the heat-sealed portion in the planar direction of the sheet-shaped absorbent layer. Meanwhile, at a non-heat-sealed portion of the absorbent polymer absent region, a bodily fluid easily passes through the sheet-shaped absorbent layer in the thickness direction. Therefore, appropriate adjustment of the proportion of the heat-sealed portion and the non-heat-sealed portion allows optional adjustment of the spread and permeation of a bodily fluid in the sheet-shaped absorbent layer. Partial heat-sealing may be achieved by heat-sealing the nonwoven fabric sheets in a predefined pattern.

The nonwoven fabric sheets may be joined together by the adhesive layer at the absorbent polymer absent region, at which the nonwoven fabric sheets may be further partly heat-sealed. Alternatively, the adhesive layer may not be provided at the absorbent polymer absent region, at which the nonwoven fabric sheets may be partly heat-sealed. Still alternatively, the nonwoven fabric sheets may not be partly heat-sealed at the absorbent polymer absent region, at which the nonwoven fabric sheets may be joined together by the adhesive layer.

The absorbent polymer present regions are preferably disposed intermittently in the width direction of the absorbent laminate. More preferably, both in the upper sheet-shaped absorbent layer and the lower sheet-shaped absorbent layer, the absorbent polymer present regions are disposed intermittently in the width direction of the absorbent laminate. When the absorbent polymer present regions are disposed intermittently in the width direction of the absorbent laminate, a bodily fluid easily spreads in the longitudinal direction in the sheet-shaped absorbent layer. In general, the absorbent laminate is longer in the longitudinal direction than in the width direction, and therefore, when a bodily fluid spreads in the longitudinal direction, the bodily fluid is rapidly absorbed easily by the absorbent laminate.

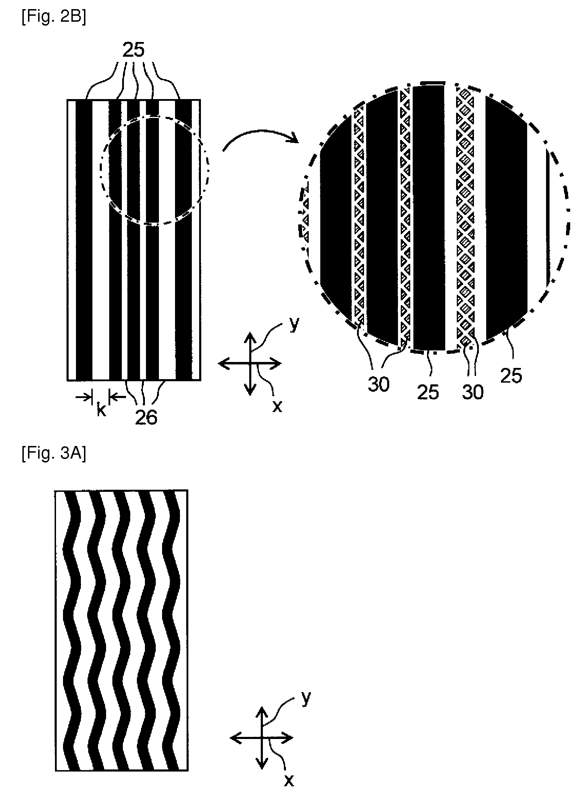

Figs. 2A, 2B and 3A to 3D show examples of an arrangement pattern of the absorbent polymer present region. In Figs. 2A, 2B and 3A to 3D, the absorbent polymer present region is expressed in black and the absorbent polymer absent region is expressed in white. In the drawings, an arrow x represents the width direction of the absorbent laminate and an arrow y represents the longitudinal direction of the absorbent laminate.

In Figs. 2A, 2B, and 3A to 3D, a plurality of the absorbent polymer present regions, in each of which the absorbent polymer is provided, and the absorbent polymer absent region adjacent to the absorbent polymer present region are provided; and the absorbent polymer present regions are disposed intermittently in the width direction x of the absorbent laminate.

In Figs. 2A and 2B, each of the absorbent polymer present regions is disposed in the shape of a practically straight line extending in the longitudinal direction y of the absorbent laminate and having a length approximately equal to the length of the sheet-shaped absorbent layer in the longitudinal direction y. When the absorbent polymer present regions are disposed in these manners, a bodily fluid easily spreads in the longitudinal direction y, and further, since the absorbent polymer present regions are disposed so as to have relatively large areas in total (e.g., when compared to Figs. 3A to 3D which are described below), the absorptive capacity of the sheet-shaped absorbent layer is easily enhanced. The widths of the plurality of absorbent polymer present regions may be same or different from each other. In addition, the plurality of absorbent polymer present regions may be disposed at regular intervals, or may not be disposed at regular intervals. For example, in Fig. 2A, the plurality of absorbent polymer present regions have the substantially same width, and are disposed at substantially regular intervals. On the other hand, in Fig. 2B, the absorbent polymer present regions in a middle portion in the width direction x are disposed at short intervals so as to have narrow widths, and the absorbent polymer present regions in side portions in the width direction x are disposed at long intervals so as to have wide widths.

In Figs. 2A and 2B, the respective absorbent polymer present regions are disposed in the shape of straight lines extending in the longitudinal direction y; however, the absorbent polymer present regions may be disposed in the shape of meandering lines extending in the longitudinal direction y as shown in Fig. 3A. Further, the absorbent polymer present region also may be disposed in the shape of a curved line extending in the longitudinal direction y, although not shown in the drawings. However, in the light of easily manufacturing the sheet-shaped absorbent layer, the absorbent polymer present region is preferably disposed in the shape of a practically straight line extending in the longitudinal direction y.

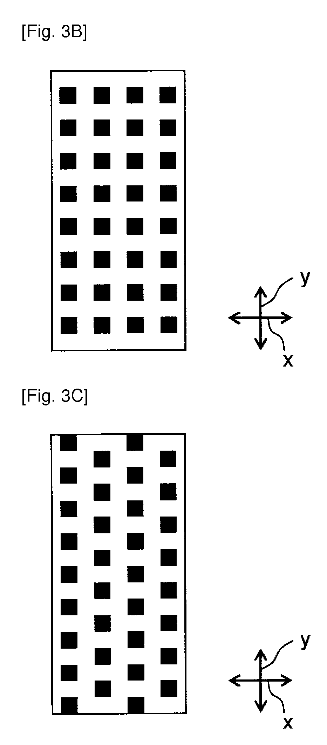

In Figs. 3B and 3C, the rectangular absorbent polymer present regions are disposed intermittently in both the width direction x and the longitudinal direction y. When the absorbent polymer present regions are disposed in these manners, a bodily fluid easily spreads in both the width direction x and the longitudinal direction y, thereby rapidly absorbed by the absorbent laminate. The rectangular absorbent polymer present regions are preferably aligned at least in the longitudinal direction y. As a result, a bodily fluid easily spreads in the longitudinal direction y. For example, in Fig. 3B, the rectangular absorbent polymer present regions are aligned in both the width direction x and the longitudinal direction y. In Fig. 3C, the rectangular absorbent polymer present regions are aligned only in the longitudinal direction y.

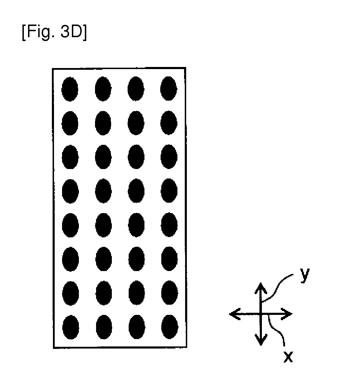

In Figs. 3B and 3C, the shapes of the absorbent polymer present regions disposed intermittently in the width direction x and the longitudinal direction y are rectangular; however, the shapes of the absorbent polymer present regions may be, for example, elliptic as shown in Fig. 3D. Further, the absorbent polymer present region also may have a circular shape, a rectangular shape whose corners are rounded, or the like, although not shown in the drawings.

Preferably, each of the absorbent polymer present regions is disposed in the shape of a practically straight line extending in the longitudinal direction and having a length of 75% or more of the absorbent laminate in the longitudinal direction, and the absorbent polymer present regions are aligned practically parallel each other in the width direction of the absorbent laminate. More preferably, in both the upper sheet-shaped absorbent layer and the lower sheet-shaped absorbent layer, the absorbent polymer present regions are disposed in this manner. When the absorbent polymer present regions are disposed in this manner, a bodily fluid easily spreads in the longitudinal direction y in the sheet-shaped absorbent layer, and further, the absorptive capacity of the sheet-shaped absorbent layer is easily enhanced since the absorbent polymer present regions are disposed so as to have relatively large areas. In addition, when the sheet-shaped absorbent layer is continuous-manufactured, it becomes easy to form the absorbent polymer present region by applying an absorbent polymer on a nonwoven fabric. The length of the linear shaped absorbent polymer present region is preferably 80% or more of the length of the absorbent laminate in the longitudinal direction, more preferably 90% or more of the length of the absorbent laminate in the longitudinal direction, and further more preferably practically equal to the length of the absorbent laminate in the longitudinal direction.

In the case that, in both the upper sheet-shaped absorbent layer and the lower sheet-shaped absorbent layer, each of the absorbent polymer present regions is disposed in the shape of a practically straight line extending in the longitudinal direction and having a length of 75% or more of the absorbent laminate in the longitudinal direction, and the absorbent polymer present regions are aligned practically parallel each other in the width direction of the absorbent laminate, it is preferable that a maximum distance between the adjacent absorbent polymer present regions of the upper sheet-shaped absorbent layer is larger than that of the lower sheet-shaped absorbent layer. In the sheet-shaped absorbent layer, the larger the distances between the absorbent polymer present regions are, that is, the larger the widths of the absorbent polymer absent regions are, the more easily a bodily fluid in the sheet-shaped absorbent layer is permeated and spread. For enhancing the permeation and spread of a bodily fluid in the sheet-shaped absorbent layer, it is more effective to dispose a few absorbent polymer absent regions having wide widths, than to dispose many absorbent polymer absent regions having narrow widths. In the absorbent article of the present invention, in order that the absorbent laminate can absorb a bodily fluid more rapidly, it is preferable to enhance the permeation and spread of a bodily fluid in the upper sheet-shaped absorbent layer rather than those in the lower sheet-shaped absorbent layer. Thus, it is preferable that the maximum distance between the adjacent absorbent polymer present regions of the upper sheet-shaped absorbent layer is larger than that of the lower sheet-shaped absorbent layer.

An example of the combination of the upper sheet-shaped absorbent layer and the lower sheet-shaped absorbent layer is the combination of a sheet-shaped absorbent layer having a pattern as shown in Fig. 2A as the upper sheet-shaped absorbent layer and a sheet-shaped absorbent layer having a pattern as shown in Fig. 2B as the lower sheet-shaped absorbent layer. Here, Figs. 2A and 2B are assumed to have the same reduced scale. In Fig. 2A, the distances between the adjacent absorbent polymer present regions are the same and have a length h. In Fig. 2B, some of the distances between the adjacent absorbent polymer present regions are larger than the others thereof, and the larger distances, that is the maximum distance, have a length k. In Figs. 2A and 2B, the maximum distance h between the adjacent absorbent polymer present regions of the upper sheet-shaped absorbent layer is larger than the maximum distance k between the adjacent absorbent polymer present regions of the lower sheet-shaped absorbent layer.

In the absorbent article of the present invention, the fiber assembly layer may have an opening for utilizing the absorption capability of the lower sheet-shaped absorbent layer more effectively. Here, the opening means an opening which penetrate the fiber assembly layer in the thickness direction. When the fiber assembly layer has an opening, a bodily fluid which has passed through the upper sheet-shaped absorbent layer can transfer directly to the lower sheet-shaped absorbent layer through the opening of the fiber assembly layer, thereby rapidly absorbed by the lower sheet-shaped absorbent layer. Therefore, the absorptive capability of the lower sheet-shaped absorbent layer is more effectively utilized. On the other hand, at a part of the fiber assembly layer where the opening is not provided, a bodily fluid which has passed through the upper sheet-shaped absorbent layer diffuses in the fiber assembly layer, and a bodily fluid which has transferred to the lower sheet-shaped absorbent layer through the opening and have spread in the planar direction is absorbed by the fiber assembly layer. As a result, the absorption rate of the absorbent laminate is increased in totally. When the fiber assembly layer has an opening, the lower sheet-shaped absorbent layer is preferably provided beneath the opening.

The fiber assembly layer may have only one opening or two or more openings. The fiber assembly layer preferably has one opening extending in the longitudinal direction of the absorbent laminate at the center in the width direction of the absorbent laminate. In this case, the opening is preferably provided at such a location that the opening crosses the center line of the absorbent laminate in the longitudinal direction. When the opening is provided in this manner, the opening comes to be located near urethral orifice of a wearer, and thus rapid absorption of a bodily fluid such as urine can be achieved.

In the case that the fiber assembly layer has only one opening, a width of the opening is preferably 10% or more of the entire width of the absorbent laminate, more preferably 20% or more of the entire width of the absorbent laminate, and a length of the opening is preferably 20% or more of the entire length of the absorbent laminate, more preferably 30% or more of the entire length of the absorbent laminate. When the opening has the width of 10% or more of the entire width of the absorbent laminate and the length of 20% or more of the entire length of the absorbent laminate, the opening is easily positioned near an urethral orifice of a wearer in wearing of the absorbent article. Meanwhile, the width of the opening is preferably 60% or less of the entire width of the absorbent laminate, more preferably 50% or less of the entire width of the absorbent laminate, and the length of the opening is preferably 70% or less of the entire length of the absorbent laminate, more preferably 60% or less of the entire length of the absorbent laminate. When the opening has the width of 60% or less of the entire width of the absorbent laminate and the length of 70% or less of the entire length of the absorbent laminate, a bodily fluid tends to be diffused well in the fiber assembly layer.

In the absorbent article of the present invention, which comprises the absorbent laminate including the upper sheet-shaped absorbent layer, the fiber assembly layer and the lower sheet-shaped absorbent layer provided in this order from the top sheet side, the fiber assembly layer is preferably longer in both the longitudinal direction and the width direction than the upper sheet-shaped absorbent layer. More preferably, the outer edge of the fiber assembly layer locates outside the outer edge of the upper sheet-shaped absorbent layer. When the upper sheet-shaped absorbent layer and the fiber assembly layer are provided in this manner, the fiber assembly layer can receive a bodily fluid which overflows from the upper sheet-shaped absorbent layer in such a case that a bodily fluid such as urine is excreted in a large amount at one time and overflow.

The size of the lower sheet-shaped absorbent layer is not particularly limited. However, if the size of the lower sheet-shaped absorbent layer is too large, the lower sheet-shaped absorbent layer is less likely to contribute wholly to absorption of a bodily fluid. Thus, the lower sheet-shaped absorbent layer is preferably shorter in the longitudinal direction and in the width direction than the fiber assembly layer. More preferably, the outer edge of the fiber assembly layer locates outside the outer edge of the lower sheet-shaped absorbent layer.

The absorbent laminate is formed by stacking the lower sheet-shaped absorbent layer, the fiber assembly layer, and the upper sheet-shaped absorbent layer. The fiber assembly layer may be joined and fixed to the upper sheet-shaped absorbent layer and the lower sheet-shaped absorbent layer by an adhesive or the like in order that the absorbent laminate is suppressed to twist or deform in using the absorbent article. Thus, adhesive layers may be provided between the upper sheet-shaped absorbent layer and the fiber assembly layer and between the fiber assembly layer and the lower sheet-shaped absorbent layer. In this case, in order that a bodily fluid smoothly transfers to a lower layer, each adhesive layer is preferably formed into a net-like structure. As an adhesive used for these adhesive layers, the adhesive which can be used in the sheet-shaped absorbent layer may be used.

The absorbent article is preferably provided with a pair of rising flaps on both sides in the width direction. Providing the rising flaps enables to prevent lateral leakage of excretion such as urine. The rising flap may be formed, for example, by joining side sheets which extend in the longitudinal direction to the top sheet on the opposite sides in the width direction, and providing elastic members to inner ends, with respect to the width direction, of the side sheets. When the side sheet and the elastic member are provided in this manner, the inner ends of the side sheets rise toward a wearer due to a shrinkage force of the elastic member to form the rising flaps. The rising flap or the side sheet is preferably made of a liquid-impermeable plastic film, a liquid-impermeable nonwoven fabric, or the like.

Elastic materials such as a polyurethane thread, a polyurethane film, a natural rubber, which are generally used for absorbent articles such as a disposable diaper, can be used for the elastic member. The elastic member is preferably fixed in a stretched state with a hot-melt adhesive. For example, a polyurethane thread having a fineness of 100 dtex to 2,500 dtex is stretched at a ratio of 1.1 to 5.0 times to be fixed. A preferable bonding means is a rubber hot-melt adhesive.

The absorbent article of the present invention can be applied to an incontinence pad, a sanitary napkin, a disposable diaper, or the like. In the case that the absorbent article is a sanitary napkin, the absorbent core is disposed between the top sheet and the back sheet, thereby forming a sanitary napkin, for example. Examples of the shape of the sanitary napkin include a substantially rectangular shape, an hourglass shape and a center nipped-in gourd shape. In the case that the absorbent article is a disposable diaper, the disposable diaper may be an open-type disposable diaper which is provided with a pair of fastening members on left and right sides of a back part or a front part and which is formed into a pants shape by using the fastening members when being worn, or the disposable diaper may be a pants-type disposable diaper in which a front part and a back part are joined to each other to form a waist opening and a pair of leg openings.

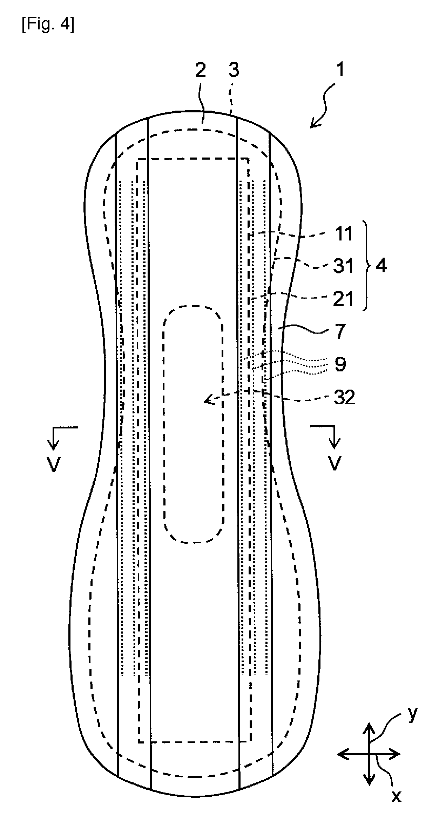

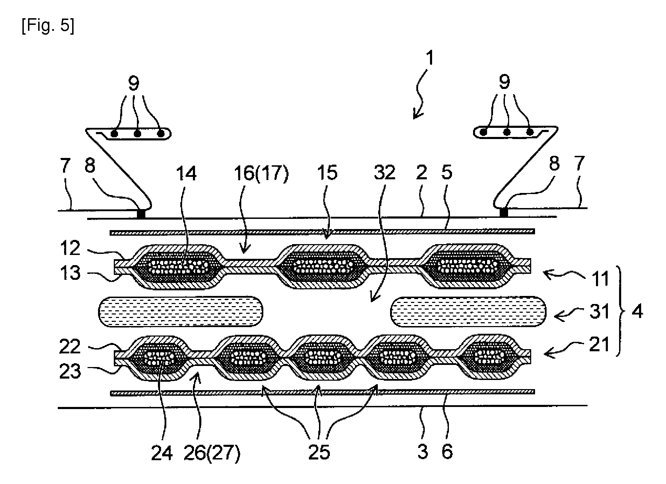

The absorbent article of the present invention is explained in the following, referring to Figs. 4 and 5, in which an incontinence pad is shown as one embodiment of the absorbent article of the present invention. Fig. 4 shows a plan view of an incontinence pad. Fig .5 shows a cross-sectional view taken along line V-V of the incontinence pad shown in Fig. 4. In the drawings, an arrow x represents the width direction and an arrow y represents the longitudinal direction. A direction on the plane formed by the arrows x and y is defined as the planar direction, and a direction orthogonal to the arrows x and y is defined as a thickness direction or a vertical direction.

An absorbent article 1 comprises a top sheet 2, a back sheet 3 and an absorbent laminate 4 disposed between the top sheet 2 and the back sheet 3. An upper base sheet 5 is provided between the top sheet 2 and the absorbent laminate 4, and a lower base sheet 6 is provided between the back sheet 3 and the absorbent laminate 4. However, in Fig. 4, the absorbent article is represented such that the upper base sheet 5 and the lower base sheet 6 are omitted.

The upper base sheet 5 and the lower base sheet 6 is provided for promoting diffusion of a bodily fluid such as urine and for preventing the absorbent laminate from loosing its shape. The upper base sheet 5 is preferably liquid-permeable, and a material which can be used for the top sheet may be used as the upper base sheet 5. The lower base sheet 6 may be liquid-permeable or liquid-impermeable, and a material which can be used for the top sheet or the back sheet may be used as the lower base sheet 6.

The top sheet 2 is placed so as to face to a wearer's skin, and allows a bodily fluid such as urine to permeate through. The bodily fluid which has passed through the top sheet 2 further permeates the upper base sheet 5, thereby transferring to the absorbent laminate 4.

Side sheets 7, which extend in the longitudinal direction y, are provided to the top sheet 2 on both sides in the width direction x. The side sheet 7 is joined to the top sheet 2 at a joining portion 8. Three rising elastic members 9 are disposed at an inner end in the width direction x of the each side sheet 7. When the disposable diaper 1 is worn, the inner end of the side sheet 7 rises toward a wearer's skin due to a shrinkage force of the rising elastic members 9, thereby preventing excrement such as urine from leaking.

The absorbent laminate 4 comprises an upper sheet-shaped absorbent layer 11, a fiber assembly layer 31 and a lower sheet-shaped absorbent layer 21 provided in this order from the top sheet 2 side. Therefore, a bodily fluid which has transferred to the absorbent laminate 4 is basically first absorbed by the upper sheet-shaped absorbent layer 11.

The upper sheet-shaped absorbent layer 11 contains an absorbent polymer 14 but does not contain a pulp fiber between nonwoven fabric sheets 12, 13. In detail, the upper sheet-shaped absorbent layer 11 has a plurality of absorbent polymer present regions 15, in each of which the absorbent polymer 14 is provided, and absorbent polymer absent regions 16 adjacent to the absorbent polymer present region 15 between the nonwoven fabric sheets 12, 13; and the nonwoven fabric sheets 12, 13 are joined together at the absorbent polymer absent regions 16 to form sealing portions 17. The absorbent polymer 14 disposed at the absorbent polymer present region 15 is fixed to the nonwoven fabric sheets 12, 13 by the adhesive layer; and the nonwoven fabric sheets 12, 13 are joined together at the absorbent polymer absent regions 16 by the adhesive layer.

A bodily fluid which has not been fully absorbed by the upper sheet-shaped absorbent layer 11 transfers to the fiber assembly layer 31. The fiber assembly layer 31 contains pulp fibers. The fiber assembly layer 31 is wider in the width direction x and longer in the longitudinal direction y than the upper sheet-shaped absorbent layer 11 and the lower sheet-shaped absorbent layer 21. In Fig. 5, the fiber assembly layer 31 has an opening 32. At the opening 32 of the fiber assembly layer 31, a bodily fluid which has passed through the upper sheet-shaped absorbent layer 11 directly transfers to the lower sheet-shaped absorbent layer 21, and is rapidly absorbed by the lower sheet-shaped absorbent layer 21. At a part of the fiber assembly layer 31 where the opening 32 is not provided, a bodily fluid which has passed through the upper sheet-shaped absorbent layer 11 diffuses in the fiber assembly layer 31, whereby the absorptive capability of the lower sheet-shaped absorbent layer 21 comes to be utilized in a broad area. As a result, the absorption rate of the absorbent laminate 4 is improved in totally.

The lower sheet-shaped absorbent layer 21 contains an absorbent polymer 24 but does not contain a pulp fiber between nonwoven fabric sheets 22, 23. In detail, the lower sheet-shaped absorbent layer 21 has a plurality of absorbent polymer present regions 25, in each of which the absorbent polymer 24 is provided, and absorbent polymer absent regions 26 adjacent to the absorbent polymer present region 25 between the nonwoven fabric sheets 22, 23; and the nonwoven fabric sheets 22, 23 are joined together at the absorbent polymer absent regions 26 to form sealing portions 27. The absorbent polymer 24 disposed at the absorbent polymer present region 25 is fixed to the nonwoven fabric sheet 22, 23 by the adhesive layer; and the nonwoven fabric sheets 22, 23 are joined together at the absorbent polymer absent regions 26 by the adhesive layer.

In Fig. 5, a cross-sectional view of the upper sheet-shaped absorbent layer 11 and the lower sheet-shaped absorbent layer 21 is shown. Meanwhile, in a planar view, the absorbent polymer present region 15 and the absorbent polymer absent region 16 of the upper sheet-shaped absorbent layer 11 are arranged in a pattern shown in Fig. 2A, and the absorbent polymer present region 25 and the absorbent polymer absent region 26 of the lower sheet-shaped absorbent layer 21 are arranged in a pattern shown in Fig. 2B. That is, the absorbent polymer absent regions 16, 26 are disposed intermittently in the width direction x of the absorbent laminate 4. Each of the absorbent polymer present regions 15, 25 is disposed in a shape of a practically straight line extending in the longitudinal direction y of the absorbent laminate 4 and having a length approximately equal to the length of the upper or lower sheet-shaped absorbent layer 11, 21 in the longitudinal direction y. The absorbent polymer present regions 15, 25 are aligned practically parallel each other in the width direction x of the absorbent laminate 4.

In the upper sheet-shaped absorbent layer 11 and the lower sheet-shaped absorbent layer 21, the nonwoven fabric sheets are partly heat-sealed at the absorbent polymer absent regions 16, 26 to form heat-sealed portions 20, 30, as shown in partially enlarged views in Figs. 2A and 2B. Therefore, a bodily fluid easily spreads in the planar direction on the upper and lower sheet-shaped absorbent layers 11,21 at the heat-sealed portion 20, 30. In Figs. 2A and 2B, each of the heat-sealed portions 20, 30 has a rhombic shape (or a partially lacked rhombic shape), and the rhombic-shaped heat-sealed portions 20, 30 are arranged in a matrix in a plane.

A maximum distance h between the adjacent absorbent polymer present regions 15 of the upper sheet-shaped absorbent layer 11 is larger than a maximum distance k between the adjacent absorbent polymer present regions 25 of the lower sheet-shaped absorbent layer 21. Therefore, permeation and spread of a bodily fluid in the upper sheet-shaped absorbent layer 11 are enhanced more, whereby the bodily fluid is more rapidly absorbed by the absorbent laminate 4.

### Reference Signs List

1: an absorbent article (an incontinence pad)
2: a top sheet
3: a back sheet
4: an absorbent laminate
11: an upper sheet-shaped absorbent layer
21: a lower sheet-shaped absorbent layer
12, 13, 22, 23: a nonwoven fabric sheet
14, 24: an absorbent polymer
15, 25: an absorbent polymer present region
16, 26: an absorbent polymer absent region
31: a fiber assembly layer
32: an opening

## Claims

1. An absorbent article (1) comprising a top sheet (2), a back sheet (3) and an absorbent laminate (4) disposed between the top sheet (2) and the back sheet (3), wherein:
the absorbent laminate (4) comprises an upper sheet-shaped absorbent layer (11), a fiber assembly layer (31) and a lower sheet-shaped absorbent layer (21) provided in this order from the top sheet side;
the fiber assembly layer (31) contains pulp fibers and has an opening (32) which penetrates the fiber assembly layer (31) in a thickness direction; and
each of the upper sheet-shaped absorbent layer (11) and the lower sheet-shaped absorbent layer (21) contains an absorbent polymer (14, 24) but does not contain a pulp fiber between nonwoven fabric sheets;
**characterized in that**:
each of the upper sheet-shaped absorbent layer (11) and the lower sheet-shaped absorbent layer (21) has a plurality of absorbent polymer present regions (15, 25), in each of which the absorbent polymer (14, 24) is provided, and an absorbent polymer absent region (16, 26) adjacent to the absorbent polymer present region (15, 25) between the nonwoven fabric sheets; and
the nonwoven fabric sheets are joined together at the absorbent polymer absent region (16, 26) to form a sealing portion.

2. The absorbent article according to claim 1, wherein the nonwoven fabric sheets of at least one of the upper sheet-shaped absorbent layer (11) and the lower sheet-shaped absorbent layer (21) are partly heat-sealed at the absorbent polymer absent region (16, 26).

3. The absorbent article according to claim 1 or 2, wherein the absorbent polymer present regions (15, 25) are disposed intermittently in a width direction of the absorbent laminate (4).

4. The absorbent article according to any one of claims 1 to 3, wherein:
each of the absorbent polymer present regions (15, 25) is disposed in a shape of a practically straight line extending in a longitudinal direction of the absorbent laminate (4) and having a length of 75% or more of the absorbent laminate (4) in the longitudinal direction; and
the absorbent polymer present regions (15, 25) are aligned practically parallel each other in the width direction of the absorbent laminate (4).

5. The absorbent article according to claim 4, wherein a maximum distance between the adjacent absorbent polymer present regions (15) of the upper sheet-shaped absorbent layer (11) is larger than that (25) of the lower sheet-shaped absorbent layer (21).

6. The absorbent article according to any one of claims 1 to 5, wherein the nonwoven fabric sheets of the upper sheet-shaped absorbent layer (11) or the lower sheet-shaped absorbent layer (21) are kept joined together at the sealing portion when the upper sheet-shaped absorbent layer (11) or the lower sheet-shaped absorbent layer (21) absorbs a bodily fluid.

7. The absorbent article according to any one of claims 1 to 6, wherein:
an adhesive is applied to the nonwoven fabric sheets of the upper sheet-shaped absorbent layer (11) and the lower sheet-shaped absorbent layer (21) respectively to form adhesive layers;
the absorbent polymer (14, 24) disposed at the absorbent polymer present region (15, 25) is fixed to the nonwoven fabric sheet by the adhesive layer; and
the nonwoven fabric sheets are joined together at the absorbent polymer absent region (15, 25) by the adhesive layer.

## Patentansprüche

1. Saugfähiger Artikel (1), der eine oberste Lage (2), eine Unterlage (3) und eine saugfähiges Laminat (4), das zwischen der obersten Lage (2) und der Unterlage (3) angeordnet ist, umfasst, wobei:
das saugfähige Laminat (4) eine obere lagenförmige saugfähige Schicht (11), eine Faseranordnungsschicht (31) und eine untere lagenförmige saugfähige Schicht (21), die in dieser Reihenfolge von der Seite der obersten Lage angeordnet sind, umfasst;
die Faseranordnungsschicht (31) Zellstofffasern enthält und eine Öffnung (32) hat, die die Faseranordnungsschicht (31) in einer Dickenrichtung durchdringt; und
jede der oberen lagenförmigen saugfähigen Schicht (11) und der unteren lagenförmigen saugfähigen Schicht (21) ein saugfähiges Polymer (14, 24) enthält, aber keine Zellstofffaser zwischen Vliesstofflagen enthält;
**dadurch gekennzeichnet, dass**:
jede der oberen lagenförmigen saugfähigen Schicht (11) und der unteren lagenförmigen saugfähigen Schicht (21) eine Vielzahl von Bereichen (15, 25) mit vorhandenem saugfähigem Polymer, in denen das saugfähige Polymer (14, 24) bereitgestellt ist, und einen Bereich (16, 26) ohne saugfähiges Polymer, der zu dem Bereich (15, 25) mit vorhandenem saugfähigem Polymer benachbart ist, zwischen den Vliesstofflagen hat; und
die Vliesstofflagen an dem Bereich (16, 26) ohne saugfähiges Polymer miteinander verbunden sind, um einen Dichtungsabschnitt zu bilden.

2. Saugfähiger Artikel nach Anspruch 1, wobei die Vliesstofflagen der oberen lagenförmigen saugfähigen Schicht (11) und/oder der unteren lagenförmigen saugfähigen Schicht (21) an dem Bereich (16, 26) ohne saugfähiges Polymer teilweise verschweißt sind.

3. Saugfähiger Artikel nach Anspruch 1 oder 2, wobei die Bereiche (15, 25) mit vorhandenem saugfähigem Polymer in einer Breitenrichtung des saugfähigen Laminats (4) intermittierend angeordnet sind.

4. Saugfähiger Artikel nach einem der Ansprüche 1 bis 3, wobei:
jeder der Bereiche (15, 25) mit vorhandenem saugfähigem Polymer in einer Form einer praktisch geraden Linie angeordnet ist, die sich in einer Längsrichtung des saugfähigen Laminats (4) erstreckt und eine Länge von 75% oder mehr des saugfähigen Laminats (4) in der Längsrichtung hat; und
die Bereiche (15, 25) mit vorhandenem saugfähigem Polymer praktisch parallel zueinander in der Breitenrichtung des saugfähigen Laminats (4) ausgerichtet sind.

5. Saugfähiger Artikel nach Anspruch 4, wobei ein maximaler Abstand zwischen den benachbarten Bereichen (15) mit vorhandenem saugfähigem Polymer der oberen lagenförmigen saugfähigen Schicht (11) größer als der (25) der unteren lagenförmigen saugfähigen Schicht (21) ist.

6. Saugfähiger Artikel nach einem der Ansprüche 1 bis 5, wobei die Vliesstofflagen der oberen lagenförmigen saugfähigen Schicht (11) oder der unteren lagenförmigen saugfähigen Schicht (21) an dem Dichtungsabschnitt miteinander verbunden gehalten werden, wenn die obere lagenförmige saugfähige Schicht (11) oder die untere lagenförmige saugfähige Schicht (21) ein Körperfluid aufsaugt.

7. Saugfähiger Artikel nach einem der Ansprüche 1 bis 6, wobei:
ein Klebstoff auf die Faserstofflagen jeweils der oberen lagenförmigen saugfähigen Schicht (11) und der unteren lagenförmige saugfähigen Schicht (21) aufgetragen wird, um Klebstoffschichten zu bilden;
das saugfähige Polymer (14, 24), das an dem Bereich (15, 25) mit vorhandenem saugfähigem Polymer angeordnet ist, durch die Klebstoffschicht an der Vliesstofflage befestigt ist; und
die Vliesstofflagen an dem Bereich (15, 25) ohne saugfähiges Polymer durch die Klebstoffschicht miteinander verbunden sind.

## Revendications

1. Un article absorbant (1) cpmprenant une feuille supérieure (2), une feuille arrière (3) et un laminé absorbant (4) disposé entre la feuille supérieure (2) et la feuille arrière (3), dans lequel :
le laminé absorbant (4) comprend une couche absorbante supérieure en forme de feuille (11), une couche d'assemblage de fibres (31) et une couche inférieure en forme de feuille (21), fournies dans cet ordre à partir du côté de la feuille supérieure ;
la couche d'assemblage de fibres (31) contient des fibres de pâte et a une ouverture (32) qui pénètre la couche d'assemblage de fibres (31) en direction de l'épaisseur; et
chacune de la couche absorbante supérieure en forme de feuille (11) et de la couche absorbante inférieure en forme de feuille (21) contient un polymère absorbant (14,24) mais ne contient pas de fibre de pâte entre des feuilles de tissu non tissé ;
**caractérisé en ce que** :
chacune de la couche absorbante supérieure en forme de feuille (11) et de la couche absorbante inférieure en forme de feuille (21) a une pluralité de régions où des polymères absorbants sont présents (15,25), dans chacune desquelles le polymère absorbant (14, 24) est disposé, et une région où des polymères absorbants sont absents (16,26) adjacente à la région où des polymères absorbants sont présents (15,25) entre les feuilles de tissu non tissé ;
les feuilles de tissu non tissé sont jointes ensembles à la région où des polymères absorbants sont absents (16, 26) afin de former une portion de scellement.

2. L'article absorbant selon la revendication 1, dans lequel les feuilles de tissu non tissé d'au moins une parmi la couche absorbante supérieure en forme de feuille (11) et la couche inférieure en forme de feuille (21) sont partiellement thermo-scellées à la région où des polymères absorbants sont absents.

3. L'article absorbant selon la revendication 1 ou la revendication 2, dans lequel les régions où des polymères absorbants sont présents (15,25) sont disposées par intermittence dans la direction de largeur du laminé absorbant (4).

4. L'article absorbant selon l'une des revendications 1 à 3, dans lequel :
chacune des régions où des polymères absorbants sont présents (15,25) est disposée dans une forme d'une ligne pratiquement droite s'étendant dans une direction longitudinale du laminé absorbant (4) et ayant une longueur de 75% ou plus par rapport à la longueur du laminé absorbant (4) dans une direction longitudinale ; et
les régions où des polymères absorbants sont présents (15,25) sont alignées pratiquement parallèles les unes aux autres dans une direction de largeur du laminé absorbant (4).

5. L'article absorbant selon la revendication 4, dans lequel une distance maximale entre les régions où des polymères absorbants sont présents (15) de la couche absorbante supérieure en forme de feuille (11) est plus grande que celle (25) de la couche absorbante inférieure en forme de feuille (21).

6. L'article absorbant selon l'une des revendications 1 à 5, dans lequel les feuilles de tissu non tissé de la couche absorbante supérieure en forme de feuille (11) ou de la couche absorbante inférieure en forme de feuille (21) sont maintenues jointes ensembles à la portion de scellement quand la couche absorbante supérieure en forme de feuille (11) ou la couche absorbante inférieure en forme de feuille (21) absorbe un fluide corporel.

7. L'article absorbant selon l'une des revendications 1 à 6, dans lequel :
un adhésif est appliqué aux feuilles de tissu non tissé de la couche absorbante supérieure en forme de feuille (11) et de la couche absorbante inférieure en forme de feuille (21) afin de former respectivement des couches adhésives ;
le polymère absorbant (14,24) disposé à la région où des polymères absorbants sont présents (15,25) est fixé à la feuille de tissu non tissé par la couche adhésive ; et
les feuilles de tissu non tissé sont jointes ensemble à la région où des polymères absorbants sont absents (15,25) par la couche adhésive.
